# EUROPEAN PATENT SPECIFICATION

(11) **EP 4 176 852 B1**
(45) Date of publication and mention of the grant of the patent: **12.08.2026**
(21) Application number: 20944236.7
(22) Date of filing: 06.11.2020
(51) Int. Cl.: A61F 2/95, A61F 2/966, A61F 2/90

(54) **STENT DELIVERY SYSTEM FOR USE IN AORTA**
STENTEINBRINGUNGSSYSTEM ZUR VERWENDUNG IN DER AORTA
SYSTÈME DE POSE D'ENDOPROTHÈSE À UTILISER DANS L'AORTE

(30) Priority: 06.07.2020 CN 202010642752
(43) Date of publication of application: 10.05.2023
(73) Proprietor: Shanghai Flowdynamics Medical Technology Co., Ltd, Shanghai 201615 (CN)
(72) Inventor: DING, Jian, Shanghai 201615 (CN); FAN, Chenying, Shanghai 201615 (CN)
(74) Representative: Gevers Patents
(86) International application number: PCT/CN2020/127161
(87) International publication number: WO 2022/007279

(56) References cited:
- WO-A1-2012/096687
- CN-A- 1 479 597
- CN-A- 102 499 801
- CN-A- 106 580 530
- CN-A- 110 353 866
- CN-U- 203 885 667
- CN-U- 209 347 136
- US-A1- 2001 056 299
- US-A1- 2017 079 820
- US-A1- 2019 223 879

## Description

### TECHNICAL FIELD

The present application relates to a stent delivery system, and in particular to a stent delivery system for use in the treatment of aortic lesions, such as aortic aneurysms or aortic dissections.

### BACKGROUND

A wall of an arterial blood vessel is composed of intima, tunica media and adventitia which are in tight contact with one other. When an inner wall of the arterial blood vessel is partially damaged, the tunica media of the wall of the arterial blood vessel is gradually peeled off under a strong impact of arterial blood flow, so that blood enters between the tunica media and the adventitia of the wall of the blood vessel to form two lumens, i.e., a true lumen and a false lumen. Aortic dissection is most commonly seen. The aortic dissection causes the arterial wall to become thin and weak, with a risk of rupture at any time. Once the dissection is ruptured, the patient will die in several minutes.

The aortic dissection is divided into two types, i.e., type A and type B (i.e., Stanford typing) according to a tearing site and an extension range of the intima. Type A refers to a lesion involving an ascending aorta, and a peeling site of the arterial wall starts from the ascending aorta, and may also occur at a proximal end of an aortic arch or of a descending aorta but involves the ascending aorta. Type B means that a peeling site of the arterial wall occurs at the descending aorta without exceeding a proximal end of an opening of a left subclavian artery.

The aortic aneurysm is also a disease of abnormal expansion of an aorta. Rupture of the aortic aneurysm is also fatal for the patient.

Therefore, it is very necessary to early diagnose and timely treat the aortic dissection and the aortic aneurysm.

A conventional solution is an EVAR intraluminal intervention method, i.e., a covered stent implantation. This solution has advantages of small trauma, fast recovery and low mortality rate. However, a site where a stent is placed often involves an aortic arch and/or an abdominal aorta, three branch artery blood vessels at a convex side of the aortic arch, and main branch artery blood vessels such as a left renal artery, a right renal artery, a coeliac trunk artery, a superior mesenteric artery, or the like at the abdominal aorta cannot be obstructed during the whole surgery and after the surgery. Conventional practice is to drill a hole at a site of the covered stent corresponding to an important branch artery. On one hand, difficulty of surgery is increased, and the surgery needs to be implemented by a surgeon with rich experience; and on the other hand, once positioning of the stent is not accurate during placement, or the stent is displaced during release, an important branch blood vessel may be obstructed to induce serious consequences. Furthermore, the stent is modified before surgery, and the manufacturer thereof may refuse to provide a warranty service for this reason.

Recently, there is also proposed a solution in which total aortic intraluminal intervention is performed by using a dense mesh stent (i.e., TEVAR intervention method). Unlike the EVAR intraluminal intervention method, this solution does not use a mechanism of mechanically obstructing a false lumen, and the dense mesh stent does not significantly hinder passage of blood flow. Instead, by forming obstruction of blood flow at an inner wall of a lesion blood vessel, hemodynamics in the false lumen are changed, the pressure in the false lumen is reduced, and intraluminal thrombus is promoted, so as to achieve the purpose of treatment. Compared with the EVAR intraluminal intervention method, since this solution uses the dense mesh stent, it not only has advantages of small trauma, fast recovery and low mortality rate, but also the dense mesh stent does not significantly obstruct delivery of blood flow to a branch artery, thereby greatly reducing difficulty of surgery. Therefore, a doctor with ordinary experience may implement this solution.

However, a treatment effect of this type of stent is not ideal enough. Since a tear of the inner wall of the blood vessel is not completely obstructed, an ideal intraluminal thrombus cannot be always formed. Furthermore, existing dense mesh stents are still difficult to be applied to all lesion types of aortic dissection and aortic aneurysm, especially difficult to be applied to type A aortic lesion.

Type A aortic dissection involves the ascending aorta, and an inner diameter of the blood vessel at this site significantly increases due to lesion, usually up to 38 mm to 55 mm. After such dense mesh stent with a large diameter is radially compressed to a delivery configuration with a very small diameter, a frictional force between the stent and a wall of a delivery catheter is significantly increased usually due to a large self-expanding force of the stent, which leads to the need for a greater thrust to release the stent. The increase of a release force of the stent makes it difficult to control the accuracy of a minimally invasive surgical implemented by remote manipulation. This increased frictional force especially makes it difficult to release the stent at a site of the ascending aorta. Since an aortic blood vessel turns almost 180° at the aortic arch, a stent delivery system entering the ascending aorta from a distal end of the aorta also turns almost 180°. When the stent is initially released, a direction of pushing force applied by a side of an operator also changes by approximately 180° when the pushing force reaches a most front end of the delivery system in the ascending aorta, which makes the operator more uncontrollable in releasing the stent in the ascending aorta, thereby increasing difficulty and risk of surgery.

WO2012096687A1 discloses a pusher assembly for a stent delivery device. The pusher assembly includes a distal end of an elongate inner member and a stent-engaging member having a proximal end and a distal end. The proximal end of the stent-engaging member is at least partially inside the distal end of the elongate inner member. The stent-engaging member includes a portion that radially outwardly extends towards the distal end of the stent-engaging member. The stent-engaging member is configured to move a stent when distally advanced and is configured to not move a stent when proximally retracted.

### SUMMARY

The invention is a stent delivery system comprising a delivery catheter and a stent as defined in claim 1.

In view of this, a main purpose of the present application is to provide a stent delivery system capable of solving or improving at least one of the above problems in the related art. Specifically, a purpose of the present application is to provide a stent delivery system intended to treat the aortic dissection or aortic aneurysm, especially type A aortic lesion. The stent delivery system has a reduced stent initial release force, and partially axial compression and/or stretching is performed on a partially released stent, to provide different radial support forces and/or fluid permeability at different segments of different treatment sites.

To this end, a first aspect of the present application provides a stent delivery system, including a delivery catheter and a stent.

The delivery catheter includes an outer catheter, an inner catheter and a push rod which are coaxially arranged sequentially from exterior to interior of the delivery catheter.

The outer catheter is provided with a proximal end and a distal end, and is provided with a first hollow cavity penetrating through the outer catheter.

The inner catheter extends in the first hollow cavity at the distal end of the outer catheter, and is provided with a second hollow cavity penetrating through the inner catheter.

The push rod extends through the first hollow cavity of the outer catheter and the second hollow cavity of the inner catheter, and extends out of a distal end of the inner catheter.

The stent is a stent for use in the treatment of aortic lesion, and is releasably maintained in the first hollow cavity between the outer catheter and the push rod at the proximal end of the outer catheter in a delivery configuration.

The delivery catheter is configured so that the outer catheter, the inner catheter and the push rod are axially movable relative to one another. A proximal end of the stent is removably constrained to a proximal end of the push rod, and a distal end of the stent is removably constrained to a proximal end of the inner catheter. The stent is a stent which is at least partially compressible and extendable along an axial direction of the stent in a natural release state, the stent has a radial support force of 100 N to 600 N and a metal coverage of 30% to 90% in the natural release state, and the stent has a radial support force of 400 N to 1000 N and a metal coverage of 80% to 95% in a release and axial maximum compression state.

The outer catheter has an outer diameter of about 5 mm to about 10 mm.

The stent is formed by weaving at least two kinds of first wires and second wires with different diameters, here each of the first wires has a diameter of 20 µm to 150 µm and each of the second wires has a diameter of 150 µm to 800 µm.

The stent is further provided with at least one sparse mesh area formed only of the second wires, and the at least one sparse mesh area is arranged at a site of a corresponding treatment site having a branch artery after the stent is released.

The stent has the same diameter, or the stent has a variable diameter, and the stent has the diameter ranging from 20 mm to 60 mm.

In the stent delivery system of the present application, one of two ends (i.e., cardiac proximal and distal ends) of the stent is removably constrained to the proximal end of the push rod, and another of the two ends of the stent is removably constrained to the proximal end of the inner catheter. According to a method for placing a stent as will be described in detail below, when the stent is released, the outer catheter is maintained stationary and the push rod is pushed in a direction toward the heart, so that the push rod may drive the stent and the inner catheter to move in the direction toward the heart, and correspondingly, the outer catheter moves in a direction away from the heart, thus the stent is released from the proximal end of the stent. Since the proximal end of the stent is constrained to the proximal end of the push rod, a frictional resistance between the stent and the outer catheter is reduced since the end of the stent is constrained during initial release. Reduction of the frictional resistance is more conducive to release of the stent, especially for release of the stent in the ascending aorta. Since the delivery catheter turns nearly 180° after passing through the aortic arch, a push direction of the push rod at a side of the operator is almost opposite to a movement direction of the push rod at a side of the ascending aorta. Therefore, the smaller a friction force between the stent and the outer catheter, the smaller a force used by the operator to push the push rod, and the easier it is for the operator to control a release process, thereby facilitating a stable release of the stent.

Furthermore, since the proximal end of the stent is constrained to the proximal end of the push rod, the stent is not fully expanded and is in a semi-release state during initial release. At this time, the diameter of the stent does not reach a diameter in a full release state thereof, and does not abut against a wall of the blood vessel, so that it is easy to adjust position of the stent. Furthermore, for example, a partially released stent may be retracted into the delivery catheter, and released again after its position is adjusted.

When at least a part of the stent is released, one end of the stent is maintained stationary, and another end of the stent is moved toward the one end (for example, the inner catheter and the outer catheter are maintained stationary, the push rod is pulled back), so that the released part of the stent may be compressed. In this way, said part has significantly increased metal coverage and radial support force, thereby achieving functions of obstructing a tear at a tearing site of an inner wall of the blood vessel and supporting a true lumen of the blood vessel. On the other hand, one end of the stent is maintained stationary, and another end of the stent is moved in a direction away from the one end (for example, the push rod is maintained stationary, the inner catheter and the outer catheter are pulled back), so that the released part of the stent may be stretched, thereby maintaining a smooth blood flow at a site such as a site having a branch artery.

One of the two ends of the stent may be temporarily fixed to the push rod by a stopper, and another of the two ends of the stent may be temporarily fixed to the inner catheter by a stopper. After the stent is properly released, the stoppers are removed from the stent finally, so that the two ends of the stent are released to complete placement of the stent.

Components configured to constrain two ends of the stent are not specifically limited in the present application, and may be any existing component which may be removed finally to release the two ends of the stent.

The stent placed at a treatment site by using the stent delivery system of the present application has different degrees of compression in a length direction thereof, so that the stent may have different metal coverage and radial support forces, while meeting requirements of obstructing the tear and not obstructing delivery of blood flow to a branch blood vessel.

The stent delivery system of the present application may have a conventional outer diameter, thereby facilitating passing through a femoral artery to place the stent a site of the aorta needing to be treated. According to the present application, even as to a stent to be released in the ascending aorta, the stent may also be assembled in the delivery catheter by axially extending and radially compressing the stent to a suitable diameter.

According to an embodiment, the outer catheter of the stent delivery system of the present application may have the outer diameter of about 5 mm to about 7 mm.

According to an embodiment, the proximal end of the stent is removably constrained to the proximal end of the push rod by a stopper, and the distal end of the stent is removably constrained to the proximal end of the inner catheter by a stopper.

The push rod is hollow for the passage of guide wires.

The stent delivery system of the present application is suitable for delivery of a flow diverter bare stent.

When the above stent is used, the delivery system of the present application may partially axially compress the stent. When the stent is released in the blood vessel, a required performance of liquid impermeability is obtained substantially in a partial segment of a non-branch blood vessel of the stent by partially axial compression, to obstruct the tear and achieve a radial support force of expanding the true lumen. In particular, at a tearing site of intima of the blood vessel, more particularly in the ascending aorta, a partially enhanced radial support force and a significantly reduced fluid (blood) permeability may be obtained by axially compressing the partially released stent. On the other hand, at a site where a branch artery is present, meshes of the stent may become sparse through partially axial extension if necessary, thereby facilitating delivery of blood to the branch artery, and facilitating placement of a branch stent through the meshes if necessary. Furthermore, due to the above characteristics of the stent, density of the woven wires may be significantly reduced after the stent is axially extended, and then the stent is radially compressed, so that the stent is easily compressed into a delivery configuration with a suitable diameter, and may be conveniently assembled into the delivery system of the present application. This is conducive for the stent delivery system of the present application to have the suitable diameter, to easily implement placement of the stent through the femoral artery.

The stent is formed by weaving at least two kinds of wires with different diameters. On one hand, this enables a large-sized stent used in the aorta to have a moderate radial support force and metal coverage, and to be radially compressed to a suitable delivery dimension. On the other hand, the thicker second wires form a basic skeleton and perform a function of providing a basic radial support force, and the thinner first wires may effectively fill gaps between the second wires and perform functions of assisting support and maintaining the shape of the stent. In particular, after the stent is axially compressed, fluid permeability of the compressed part may be significantly reduced due to presence of the first wires, to form effective obstruction on the tearing site of the inner wall of the blood vessel. In addition, after the thicker second wires are axially compressed, density of the second wires per unit length is increased, so that a significantly enhanced radial support force may be formed, thereby perform a function of expanding the true lumen of the blood vessel and helping to reduce the false lumen.

In the range of the radial support force in the natural release state as defined above, the stent of the present application may be easily assembled into a delivery system with a suitable outer diameter (for example, 5 mm to 10 mm) even though the stent has a maximum diameter (such as 38 mm to 60 mm) suitable for the ascending aorta. Furthermore, after the stent is maximum compressed axially, the stent has a radial support force in the above range, so that it may effectively support a narrowed true lumen of the blood vessel. In particular, a radial support force in the preferred range is particularly conducive to a lesion site which has been formed for a certain period of time, where a torn intima of the blood vessel becomes hard and requires a greater force to be expanded.

Therefore, in an actual application, when the stent of the present application is released in the blood vessel (especially the aorta), the stent has different compression ratios in a length direction thereof, and radial support forces of the stent at different sites may vary in a range of greater than or equal to 100 N, preferably a range of 100 N to 1000 N.

According to an embodiment for reference only, the stent has a substantially complete liquid permeability in the natural release state, that is, the stent does not form obstruction of blood flow from the aorta to the branch artery substantially or completely. According to the embodiment for reference only, the stent has a radial support force of 200 N to 600 N and a metal coverage of 30% to 60% in the natural release state. The stent having a radial support force and a metal coverage in above specified ranges has a relatively low radial support force and a relatively low metal coverage in the natural release state, but has a relatively large compressible ratio. The stent may still form necessary radial support force and metal coverage meeting the above provisions after it is compressed, to effectively obstruct a tear of the inner wall of the blood vessel.

According to another embodiment for reference only, the stent has a radial support force of 100 N to 600 N and a metal coverage of 60% to 90%, preferably a metal coverage of 70% to 90%, in the natural release state. The stent having a radial support force and a metal coverage in above specified ranges has a relatively high radial support force and a relatively high metal coverage in the natural release state, but has a relatively large extendable ratio, and the stent may still be radially compressed to a suitable dimension after it is extended, to be assembled into a suitable delivery system. The stent has a relatively large radial support force and a relatively large metal coverage without compression after it is partially released, thereby facilitating immediate support and expansion of the true lumen of the blood vessel, and obstructing the tear to a certain extent. Further, the radial support force may be further enhanced and the metal coverage may be increased by axially compressing a partial segment of the stent, to form necessary radial support force and metal coverage meeting the above provisions, so as to obstruct the tear of the inner wall of the blood vessel more effectively.

The stent has a certain liquid permeability in the natural release state, that is, the stent obstructs blood flow from the aorta to the branch artery to some extent. In a specific embodiment, since the stent according to the present application is axially extendable, a suitable blood permeability may be obtained by properly stretching a partial segment corresponding to the branch vessel when the stent is placed by using the delivery system of the present application. To this end, a corresponding part of the stent is configured to have an increased diameter relative to an adjacent part thereof, so that the diameter of this area after it is stretched is suitable for an inner diameter of a lumen of the blood vessel having a branch blood vessel. The stent is provided with at least one sparse mesh area formed only of the second wires, and the at least one sparse mesh area is arranged at a site of a corresponding treatment site having a branch artery after the stent is released. According to one solution, the treatment site includes an aortic arch, and the sparse mesh area includes a first sparse mesh area. The first sparse mesh area corresponds to a greater curvature of the aortic arch, especially corresponds to a branch artery on the aortic arch: a brachiocephalic trunk artery, a common left neck artery, and a left subclavian artery. Specifically, a length of the first sparse mesh area is greater than a length of the branch arterial area on the aortic arch, such as 6 cm to 8 cm, and the first sparse mesh area corresponds to a circumferential angle of about 120° to about 180°. According to another solution, the treatment site includes an abdominal aorta, and the sparse mesh area includes a second sparse mesh area. The second sparse mesh area corresponds to a branch artery part of the abdominal aorta, especially corresponds to a branch artery on the abdominal aorta: a left renal artery, a right renal artery, a coeliac trunk artery, and a superior mesenteric artery. Specifically, a length of the second sparse mesh area is greater than a length of the branch arterial distribution area on the abdominal aorta, such as 2 cm to 4 cm, and the second sparse mesh area corresponds to a circumferential angle of about 180°. In different solutions, the stent may be provided with the first sparse mesh area and/or the second sparse mesh area.

In the embodiment, any mesh of each sparse mesh area is expandable, allowing the branch stent to be placed through the expanded mesh.

The stent may be woven according to requirements of the metal coverage of other parts of the stent, and after weaving is completed, the first wires are removed in an area where the sparse mesh area is predetermined to be formed, to form the sparse mesh area.

According to an embodiment, the stent may be used in an aorta including an abdominal aorta site, and the stent may be internally provided with two common iliac artery stent fixing parts configured to fix left and right common iliac artery stents.

According to a specific embodiment, the common iliac artery stent fixing parts may be arranged inside the stent and correspond to the abdominal aorta close to a bifurcation of left and right common iliac arteries, and the two common iliac artery stent fixing parts may be configured as two annuluses tangent to each other and may be integrally formed with an inner wall of the stent.

Unlike fixing parts forming two cylindrical branches at a lower part of a stent for the abdominal aorta, the two fixing parts of the stent of the present application are arranged inside the stent, so that the exterior of the stent is still maintained to be cylindrical, which is more conducive to expanding the blood vessel, without poor support near the common iliac arteries. Furthermore, the common iliac artery fixing parts are integrally formed with the stent, to fix left and right iliac artery stents more stably.

According to an embodiment for reference only, each of the first wires for the stent may have a diameter of 50 µm to 150 µm; and each of the second wires may have a diameter of 150 µm to 600 µm.

According to an embodiment, the stent is formed by weaving three kinds of wires with different diameters, here the second wires may include at least one first thick wire and at least one second thick wire with different diameters. According to a specific embodiment, the at least one first thick wire may have a diameter of 150 µm to 300 µm, and the at least one second thick wire may have a diameter of 300 µm to 600 µm.

According to another embodiment, the stent is formed by weaving three kinds of wires with different diameters, here the first wires may include at least one first thin wire and at least one second thin wire with different diameters. According to a specific embodiment, the at least one first thin wire may have a diameter of 20 µm to 100 µm, and the at least one second thin wire may have a diameter of 100 µm to 150 µm.

According to still another embodiment for reference only, the stent is formed by weaving four kinds of wires with different diameters, i.e., the first and second thin wires as well as the first and second thick wires.

The number of wires for weaving the stent may be 48 to 202, preferably 64 to 196. Here the number of the second wires may be 4 or more, such as 4 to 32, preferably 4 to 30, and the remaining wires are the first wires.

According to an embodiment for reference only, the second wires include 6 to 24 first thick wires and at least four second thick wires, and the remaining wires are the first wires.

In case of too much number of thick wires (i.e., the second wires), the stent cannot be effectively compressed into an ideal delivery state, while in case of too small number of thick wires, the stent cannot provide an expected radial support force even after compression, and an expected structure and morphology of the stent cannot be maintained in a release state. In particular, as to the stent of the present application, the number of the second wires preferably is less than or equal to 24.

According to an embodiment, the stent may be formed by weaving 48 to 202 wires, in which 6 to 24 first thick wires and 4 to 24 second thick wires of the 48 to 202 wires form the second wires, and remaining wires of the 48 to 202 wires form the first wires, provided that a sum of a number of the first thick wires and a number of the second thick wires is less than or equal to 32.

According to an embodiment, the first wires may include wires with two diameters. In the embodiment, the number of wires for weaving the stent may be 48 to 202. Here the number of the second wires may be 4 to 32, preferably 4 to 30, and the remaining wires are the first wires. The first wires may include 32 to 166 first thin wires and 32 to 166 second thin wires, provided that a sum of the number of the first thin wires and the number of the second thin wires is less than or equal to 198.

According to an embodiment for reference only, in order to obtain a greater metal coverage and/or a greater radial support force, the stent may be provided with at least two layers of woven meshes. According to an embodiment for reference only, the stent is provided with two to four layers of woven meshes. When the stent is provided with multiple layers of woven meshes, the stent as a whole has the radial support force and the metal coverage as specified above.

A method for weaving the stent of the present application is not specifically limited. A conventional overlapping and weaving may be used (that is, there is no constraint at an intersection between the wires), as long as it is possible to facilitate axial compression and stretching of the stent.

It should be understood that according to a range of the radial support force and a range of the metal coverage required for a specific treatment type, those skilled in the art may select a suitable woven material and determine a reasonable number of layers under specific device conditions according to descriptions herein, further select a suitable diameter, number, or the like of the wire, and determine a suitable weaving solution (such as weaving density) to obtain a stent having the required range of the radial support force and the required range of the metal coverage. For example, a suitable weaving solution may be designed by a dedicated software.

According to a further embodiment for reference only, an end (especially the proximal end where the stent has a maximum radial support force) of the stent may be formed in a return weaving manner. As for another end of the stent, if there is a burr which cannot be woven again in a return weaving manner, the burr may be located on an inner side of the stent by selecting a suitable arrangement of the layers; or if there are two layers of burrs, the burr at one layer of the two layers is woven in a return weaving manner by a short segment, and the other burr is wrapped in said segment which is woven in a return weaving manner, or when multiple stents are used in cooperation, burrs at a single layer or two layers may be overlapped with each other and placed in another stent. The stent has a smooth end, thereby avoiding mechanical damage to the inner wall of the blood vessel by an exposed end (burr) of the wire.

According to an embodiment for reference only, the stent may have the diameter ranging from 20 mm to 55 mm.

According to an embodiment for reference only, the stent of the present application in a release state is provided with a first segment arranged at the proximal end of the stent. The first segment may have a diameter of 38 mm to 60 mm to be suitable for the ascending aorta. The first segment has a length of 8 cm to 11 cm. The stent may be further provided with a second segment adjacent to the first segment. The second segment may have a diameter of 25 mm to 35 mm to be suitable for a part of the artery from the aortic arch to the abdominal aorta. Following the first segment, the second segment extends to the distal end of the stent. The second segment may have a length of 25 cm to 45 cm, preferably 25 cm to 31 cm.

According to another embodiment for reference only, the stent may have a diameter of 20 mm to 30 mm to be suitable for a part of the abdominal aorta. The stent may have a length of 15 cm to 25 cm, preferably 15 cm to 20 cm.

The stent of the present application is self-expandable or capsular expandable. Materials of the first wires and materials of the second wires for weaving the stent may be different from each other, however, preferably the same. Usually, the materials of the wires may be metal, such as stainless steel, shape memory alloy (e.g., nitinol), cobalt-chromium alloy, tungsten, or tantalum.

### BRIEF DESCRIPTION OF THE DRAWINGS

FIG. 1 is a schematic view of a stent delivery system according to the present application.
FIG. 2 is a schematic view of a stent delivery system of the present application in a partial release state.
FIG. 3 is a schematic view illustrating partially axial compression and stretching of a stent according to the present application.
FIG. 4 is a schematic view illustrating a process of placing a stent in an aorta with a tear according to an embodiment for reference only by using a stent delivery system of the present application.
FIG. 5 is a schematic view illustrating placing a stent in a type A aortic dissection lesion by using a stent delivery system of the present application.
FIG. 6 is a schematic view and partially enlarged view of a stent applicable to an embodiment of a stent delivery system of the present application.
FIG. 7 is a schematic view of a stent applicable to another embodiment of a stent delivery system of the present application.
FIG. 8 is a schematic view of a stent applicable to yet another embodiment of a stent delivery system of the present application.
FIG. 9 is a schematic partially enlarged view of a stent applicable to still another embodiment of a stent delivery system of the present application.
FIG. 10 is a schematic cross-section view of a woven layer of a stent with multiple layers according to the present application.

### DETAILED DESCRIPTION

Technical solutions in embodiments of the present application will be clearly and completely described below in combination with the embodiments of the present application and the drawings. It is apparent that the described embodiments are only part of the embodiments of the present application, rather than all the embodiments, and the technical solutions recited in the embodiments of the present application may be implemented in any combination without conflict. All other embodiments obtained by those of ordinary skill in the art based on the embodiments of the present application without paying any creative work belong to the scope of protection of the present application.

Throughout the description, terms used here should be understood as meanings as usually used in the art, unless specifically stated otherwise. Therefore, unless defined otherwise, all technical and scientific terms used here have same meanings as usually understood by those skilled in the art to which the present application belongs. When there is a contradiction, meanings of the description are preferred.

Like reference numerals in the drawings refer to like components. Shapes and dimensions of components in schematic drawings are for illustration only and cannot be considered to reflect actual shapes, dimensions and absolute positions.

It should be noted that in the present application, terms "including", "include", or any other variants thereof are intended to cover a non-exclusive inclusion, so that a method or device including a series of elements not only includes elements which are explicitly recited, but also includes other elements which are not explicitly listed, or further includes elements inherent to implementation of the method or device.

It should be noted that terms "first \ second" involved in the present application are only intended to distinguish similar objects, and do not represent a specific sequence of the objects, and it may be understood that "first \ second" may exchange a specific sequence or order in an allowable situation. It should be understood that objects distinguished by "first \ second" may be interchanged in an appropriate situation, to enable embodiments of the present application described here to be implemented in an order other than those illustrated or described here.

In order to describe the present application more clearly, terms "proximal end" and "distal end" are customary terms in the field of intervention medical treatment. Here "distal end" represents an end away from the heart during surgical operation, and "proximal end" represents an end close to the heart during surgical operation.

The present application provides a stent delivery system. In the delivery system, a stent for use in the treatment of aortic lesion is assembled in the system in a delivery configuration, and both ends of the stent are constrained. The constraints are removed only after other parts of the stent are released, so that the stent is completely released.

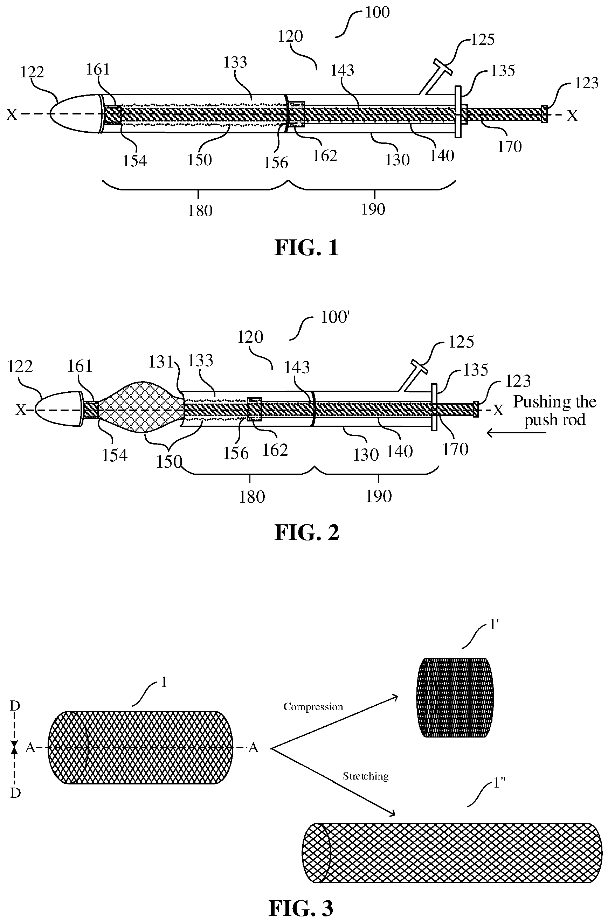

With reference to FIG. 1, a schematic view of a stent delivery system 100 according to the present application is shown. The stent delivery system 100 includes a delivery catheter 120 and a stent 150.

The delivery catheter 120 includes an outer catheter 130, an inner catheter 140 and a push rod 170 which are coaxially arranged sequentially from exterior to interior of the delivery catheter along a longitudinal axis X-X. The delivery catheter 120 is provided with a distal end 123 and a proximal end 122. The delivery catheter 120 is further provided with a hemostatic valve 125. The outer catheter 130 is provided with a proximal end 180 and a distal end 190, and a first hollow cavity 133 penetrates through the whole outer catheter 130. The inner catheter 140 is arranged coaxially with the outer catheter 130 along the longitudinal axis X-X in the first hollow cavity 133 at the distal end 190 of the outer catheter, and the inner catheter 140 is provided with a second hollow cavity 143. The push rod 170 extends through the first hollow cavity 133 of the outer catheter 130 and extends through the second hollow cavity 143 of the inner catheter 140, until extending beyond a port 135 of the distal end of the outer catheter. The push rod 170 may be provided with a third hollow cavity (not shown) for the passage of guide wires.

At the proximal end 180 of the outer catheter 130, the stent 150 is releasably maintained in the first hollow cavity 133 between the push rod 170 and the outer catheter 130 in a delivery configuration. A proximal end 154 of the stent 150 is constrained at a proximal end of the push rod 170 by a first constraint component 161. The first constraint component 161 may be a conventional stopper which may be removed from the stent 150 if necessary, to release the proximal end 154 of the stent 150. A distal end 156 of the stent 150 is constrained at a proximal end of the inner catheter 140 by a second constraint component 162. Similarly, the second constraint component 162 may be a conventional stopper which may be removed from the stent 150 if necessary, to release the distal end 156 of the stent 150.

With further reference to FIG. 2, the stent delivery system shown in FIG. 1 in a semi-release state is shown. In FIG. 2, a proximal end 131 of the outer catheter 130 in the delivery system 100' may be separated from the proximal end 122 of the delivery catheter 120, the outer catheter 130 may move relative to the inner catheter 140 and the push rod 170 by maintaining the outer catheter stationary and pushing the push rod 170 toward the proximal end (or by maintaining the inner catheter and the push rod stationary and pulling the outer catheter 130 toward the distal end).

In the system 100' shown in FIG. 2, by pushing the push rod 170 toward the proximal end 122, the proximal end 122 of the delivery catheter 120 drives the push rod 170 fixedly connected to the proximal end 122, the stent 150 constrained together with an end of the push rod 170 and the inner catheter 140 constrained together with the stent 150 to move toward the proximal end relative to the outer catheter 130. In this way, the stent 150 is started to be released from the proximal end 154 thereof.

Since the proximal end 154 of the stent 150 is constrained during release, the diameter of the released segment does not reach the diameter thereof in a natural release state thereof, and the released segment is not in contact with a wall of the blood vessel. On one hand, it is conducive to adjusting the position of the stent, and on the other hand, the stent 150 may also be retracted into the outer catheter 130 again, and the stent 150 is released again after its position is adjusted. Furthermore, the proximal end 154 of the stent 150 is constrained, which is also conducive to reducing a friction force during initial release and conducive to a stable release. This is particularly important for surgery in which distal operations are performed in a narrow blood vessel.

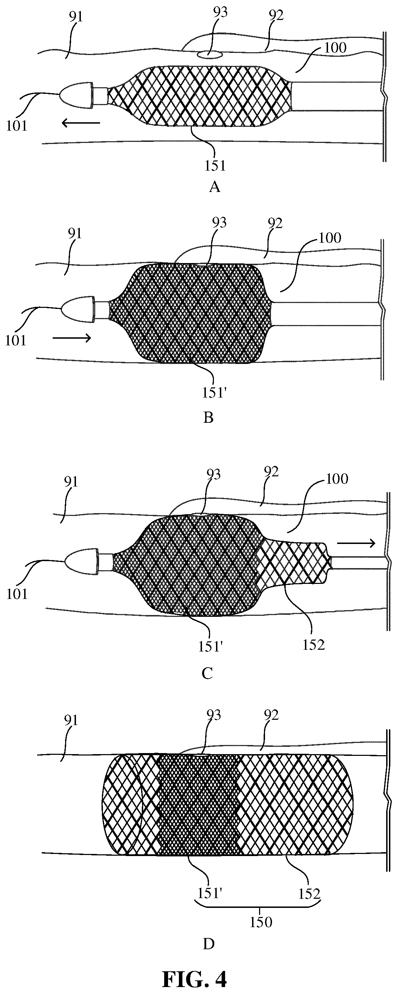

A method for releasing a stent in an aorta by using the stent delivery system of the present application is described in further detail in combination with FIG. 3 and FIG. 4.

The present application provides a method for placing a stent by using the stent delivery system of the present application, which includes the following operations.

The stent delivery system according to the present application is guided to a treatment site.

At least a part of the stent is released.

One end of the released part of the stent is maintained stationary, and the stent delivery system is manipulated, so that another end of the released part moves along an axial direction of the stent to compress/extend the released part.

After the stent is completely released, constraints on two ends of the stent are removed.

According to the method for placing the stent, the position of the stent is adjusted after the stent is released for a certain length, and the push rod may be pulled back in a direction away from the heart when the position of the inner catheter and the position of the outer catheter are stationary for example (or, the inner catheter and the outer catheter are pushed in a direction close to the heart when position of the push rod is stationary), so that the semi-released stent is driven to be compressed axially. Such axial compression is caused due to the fact that in the stent delivery system of the present application, two ends of the stent are constrained, so that when one of the two ends is stationary and another end of the two ends is moved, the released part of the stent may be axially compressed/stretched. With the stent delivery system of the present application, the released segment of the stent may be compressed to have its maximum metal coverage and maximum radial support force, so that it tightly abuts against a wall of the blood vessel at a corresponding site (especially a tearing site of the intima) under the action of its radial support force, to complete release of this segment.

Similarly, other parts of the stent may be selected to be completely released directly as desired, or may be released while compressed/stretched in segments. After the release substantially completes finally, constraints on the two ends of the stent are removed, and the delivery catheter together with the push rod is withdrawn from the lumen of the blood vessel.

The method for placing the stent of the present application further includes the following operations. It is detected whether a release position of the stent is correct after at least a part of the stent according to the present application is released.

When it is found that the release position is incorrect, the stent delivery system is adjusted so that the released part is located at a correct position, or the released part is retracted into the delivery catheter, the stent delivery system is adjusted to the correct position, and the stent is released again.

Since in the stent delivery system of the present application, the two ends of the stent are temporarily constrained, the stent may be compressed or stretched in segments during release of the stent. In particular, as to the tearing site of the inner wall of the blood vessel, effective obstruction and a required radial support force may be formed at this local area by compression operations to achieve a best therapeutic effect. Furthermore, other parts or specific parts of the stent do not hinder blood flow, and does not create a risk caused by interruption or deficiency of blood supply to the branch artery.

Optionally, in the branch arterial area, a larger mesh may also be formed at this local area by stretching operations, to facilitate placing the branch stent in the branch blood vessel. In the method, the operation that at least a part of the stent is released includes an operation that the sent is released from a proximal end of the stent.

In the method, the proximal end portion of the stent is compressed. Since a tear of the aortic dissection usually occurs at an end of the dissection close to the heart, the proximal end portion of the stent is usually required to be compressed, to obstruct the tear.

According to the method of the present application, the delivery catheter is withdrawn from the treatment site after placement of the stent is completed.

The method further includes the following operations. The stent delivery system is introduced from a femoral artery of the subject, and the delivery catheter is withdrawn from the femoral artery of the subject after the stent is placed.

The method further includes the following operations. The stent delivery system is guided to the treatment site by guide wires.

The subject is a mammal, preferably a pig or a person, more preferably a person, most preferably a person with yellow race.

The treatment site is any site from the ascending aorta to the abdominal aorta. The treatment site includes a tearing site of the intima of the blood vessel. The treatment site further includes a branch vessel.

The stent delivery system of the present application is configured to treat the aortic dissection or aortic aneurysm of the subject.

FIG. 3 schematically shows cases in which the stent is compressed and stretched respectively. The stent in the stent delivery system of the present application is a stent for use in the treatment of aortic lesion. The stent is at least partially compressible and extendable along an axial direction of the stent in a natural release state (i.e., without subject to axial compression and without extension).

As shown in FIG. 3, in case that one end of the stent is fixed, the stent 1 may be axially compressed along a central axis A-A of the stent 1 into a compressed stent 1', or the stent 1 may be axially stretched into a stretched stent 1". The compressed stent 1' has a significantly increased metal coverage, thereby obtaining a significantly reduced fluid permeability and a significantly increased radial support force, to be adapted to a tearing site of intima of the blood vessel. The stretched stent 1" has a significantly reduced metal coverage, so that in some cases, the stent is more readily compressed radially, to obtain a suitable delivery configuration, which is conducive to assembling a stent with a larger diameter into the stent delivery system of the present application.

The expression "axial direction" of the stent as mentioned here refers to a direction along A-A as shown in FIG. 3, which is a direction of a central axis of a cylindrical shape of the stent. The expression "radial direction" of the stent as mentioned here refers to a direction along D-D as shown in FIG. 3, which is a diameter direction of a circle of the cylindrical shape of the stent. In general, "radially compressed" here refers to compression in a direction from the circumference to the center of the circle.

The expression "natural release state" of the stent as mentioned here refers to a state in which the stent is not axially compressed or stretched and is not radially compressed in a water bath at 37 ± 2 °C.

The expression "axial maximum compression state" of the stent as mentioned here refers to a state in which the stent is axially compressed until it is unable to be further compressed, when it is in the natural release state.

Further in combination with FIG. 4 for reference only, a schematic view illustrating placing a stent at a tear of intima of an aorta by using the stent delivery system of the present application is shown. The part A of FIG. 4 shows a segment of aortic blood vessel 91 including a tear 93 of intima of the blood vessel, and tearing of the intima is induced by the tear 93 to form a false lumen 92. The stent delivery system 100 of the present application has been guided to the tear 93, and has released a segment 151 of the stent by pushing the push rod in a direction indicated by the arrow in case that the position of the outer catheter 130 remains unchanged. With further reference to the part B of FIG. 4, the position of the inner catheter and the position of the outer catheter of the delivery system 100 remain unchanged, and the push rod is pulled back in a direction indicated by the arrow in this figure, so that the released segment 151 of the stent is compressed back and finally abuts against a blood vessel area in which the tear 93 of the intima of the blood vessel is located, to form a compressed segment 151'. When the stent is designed, the diameter of the stent is usually designed to be slightly greater than the diameter of the blood vessel at a placement site. Therefore, the compressed segment 151'tightly abuts against an inner wall of this segment of the blood vessel, obstructs the tear 93, and expands a true lumen of this segment of the blood vessel. The compressed segment 151'is remained in this segment of the blood vessel with the compressed shape due to an inward contraction force caused by the wall of the blood vessel.

Next, as shown in the part C of FIG. 4, the remaining segment 152 of the stent is further released by pulling the outer catheter 130 toward the distal end in a direction indicated by the arrow in this figure (the inner catheter 140 and the push rod 170 remain stationary). Finally, after the stent is completely released, constraints of the stoppers on both ends of the stent are removed, so that the whole stent 150 is released to the treatment site, and the delivery catheter is withdrawn from the blood vessel (see the part D of FIG. 4). The stent 150 placed at a lesion site is provided with two segments, one of which is the compressed segment 151', and the other of which is a natural release segment 152. The compressed segment 151' performs functions of obstructing the tear 93 and forming relatively strong radial support on the blood vessel. The natural release segment 152 performs a function of supporting other parts of the blood vessel appropriately, and does not hinder flow of blood, in particular flow of blood toward a branch blood vessel.

According to the present application, after the segment 151 of the stent is released (i.e., a state shown in the part A of FIG. 4), the position of the stent may be confirmed, so that the tear 93 may be accurately obstructed by the compressed segment. If said position is not ideal enough, adjustment may be performed, and even the released segment 151 is retracted into the outer catheter. The stent is released again after the position of the delivery system is adjusted.

Similarly, the position of the stent may be confirmed after any segment of the stent is released, to achieve a best placement effect. Finally, the position of the stent is confirmed again before constraints on both ends of the stent are removed, since the stent may be retracted and released again when it is found that its placement position is not ideal at this time. After constraints on both ends of the stent are removed, the position of the stent cannot be adjusted.

Furthermore, other segments of the stent are compressed in a manner similar to that shown in the part B of FIG. 4. For example, when a front part of the stent is released and has abutted against the wall of the blood vessel, a segment of the stent continues to be released. Since a subsequently released end of the stent is constrained by the outer catheter, by remaining the push rod stationary and simultaneously moving the outer catheter and the inner catheter toward the proximal end, a segment, which is newly released but has not yet abutted against the wall of the blood vessel, of the stent is compressed and abuts against the wall of the blood vessel, to form a segment with a high metal coverage and a high radial support force.

The stent delivery system 100 of the present application is particularly advantageous for the treatment of type A aortic dissection.

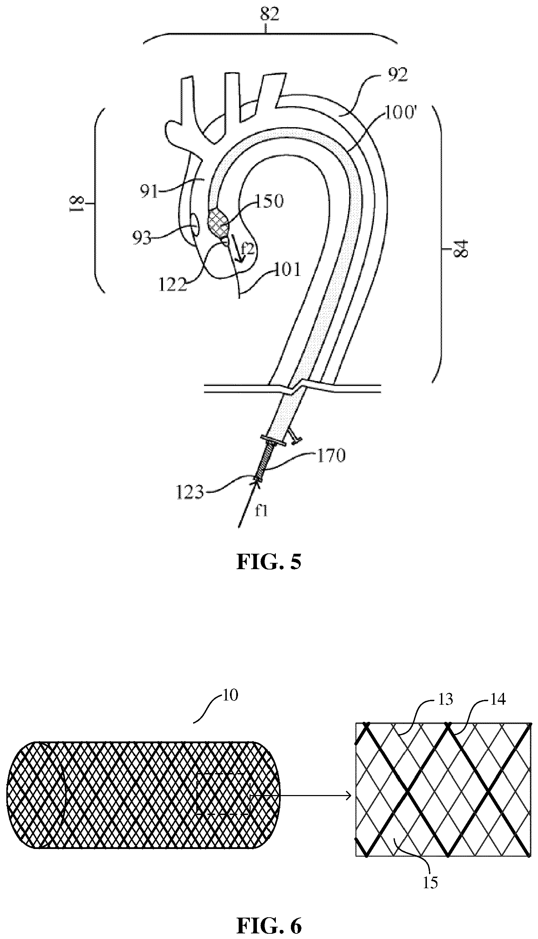

With reference to FIG. 5, a schematic view illustrating placing a stent 150 in a type A aortic dissection lesion by using the stent delivery system 100' of the present application is shown, to explain advantages of the treatment of the stent delivery system of the present application on the type A aortic dissection. FIG. 5 shows that there is a tear 93 in an ascending aorta 81, thus a false lumen 92 is formed from the ascending aorta 81 through an aortic arch 83 to a descending aorta 84. The stent delivery system 100' of the present application has been guided into a true lumen 91 of the aorta by a guide wire 101. When the stent 150 is started to be released, the operator pushes the push rod 170 in a direction toward the proximal end, where the thrust is represented by f1 and a direction of the thrust is indicated by an arrow directed upward along the descending aorta. The thrust f1 is transmitted to the proximal end 122 of the delivery catheter along the push rod 170. At this time, since the delivery system 100' turns nearly 180° after passing through the aortic arch 83, a direction of a force f2 acting on the proximal end 122 of the delivery catheter is almost opposite to the direction of the thrust at the distal end 123 of the delivery catheter (as indicated by the arrow). This causes difficulty for controlling a magnitude of the thrust required during initial release of the stent. In the stent delivery system 100' of the present application (see FIG. 1 and FIG. 2), the proximal end of the stent 150 is temporarily constrained at the proximal end of the push rod 170 by a component such as a stopper, thereby significantly reducing an outward expansion force of the stent 150 at the proximal end, and reducing the friction force between the stent 150 and an inner wall of the outer catheter 130. Therefore, the thrust required when the stent is initially released by the delivery system 100' of the present application is significantly reduced, and the proximal end of the push rod 170 may be easily pushed out of the outer catheter 130.

The stent delivery system of the present application is particularly suitable to be assembled with a stent which is at least partially compressible and extendable along an axial direction of the stent in a natural release state. Advantageously, the stent has a radial support force of greater than or equal to 100 N and a metal coverage of 30% to 90%, to provide a of delivery configuration with a suitable dimension, as well as suitable fluid permeability and radial support force after partial compression.

The stent is formed by weaving at least two kinds of first wires and second wires with different diameters, here each of the first wires has a diameter of 20 µm to 150 µm and each of the second wires has a diameter of 150 µm to 800 µm. FIG. 6 shows a schematic view and partially enlarged view of a stent 10 according to the embodiment. The stent 10 is formed by overlapping and weaving multiple first wires 13 and multiple second wires 14. The stent 10 shown in FIG. 6 is a single-layer mesh structure with meshes 15. The stent of the embodiment is more suitable for the stent delivery system of the present application. Since two kinds of wires with different diameters mainly achieve functions of reducing fluid permeability and enhancing radial support force respectively in a large-size stent for the aorta, the stent may be compressed to a suitable delivery configuration to be assembled into the stent delivery system for the aorta according to the present application, while advantages of the two aspects are obtained.

The stent may also be a structure with multiple layers, such as 2 to 4 layers. For example, the multiple layers may be formed in a return weaving manner.

The stent of the present application is suitable for any segment from the ascending aorta to the abdominal aorta or even the whole aorta. Therefore, the stent of the present application may have a large diameter ranging from about 55 mm to about 20 mm.

The stent 10 may be formed by weaving a total of 48 to 202 wires, preferably 64 to 196 wires. For example, by way of enumeration, a stent of the present application may be formed by weaving 48, 96, 128, 156, 196 wires. The number of the wires may be determined according to the diameter of the stent, the number of layers, materials of the used wires, or the like.

Material for the stent may be any material suitable for a peripheral vascular stent, as long as the material may provide a sufficient radial support force and have a certain fineness. Usually, metal wires such as stainless steel wires, nickel-titanium alloy wires, cobalt-chromium alloy wires, tungsten wires, tantalum wires, or the like are preferably used, and stainless steel wires are preferred.

There are at least four second wires 14 serving as thick wires, and usually the number of the second wires 14 is less than or equal to 30. The diameter of each second wire 14 is included between 150 µm and 800 µm, preferably between 150 µm and 600 µm. The diameter of each second wire is for example 150 µm, 200 µm, 250 µm, 300 µm, 350 µm, 400 µm, 450 µm, 500 µm, 550 µm, or 600 µm. The second wires 14 provide a basic support force and a complete structure for the stent 10. However, the number of the second wires cannot be too much. For example, even though wires with the diameter of only 300 µm are used, when about 32 wires are used, the stent is difficult to be compressed to a suitable delivery dimension and thus is unable to use, especially for a stent part with ultra-large diameter greater than about 40 mm.

In the stent 10, the remaining wires except the thick wires are first wires 13 serving as thin wires. Each first wire 13 of the present application may have a diameter of 20 µm to 150 µm, preferably a diameter of 50 µm to 150 µm, such as 50 µm, 60 µm, 70 µm, 80 µm, 90 µm, 100 µm, 110 µm, 1120 µm, 130 µm, 140 µm and 150 µm. The first wires 13 perform functions of auxiliary supporting the stent 10 and filling gaps between the second wires 14. Furthermore, the first wires 13 also perform a function of maintaining the shape of the stent 10, since the number of the first wires is much greater than the number of the second wires 14. The inventors have found that although it seems to be feasible theoretically, in fact, when the overlapping and weaving manner of the present application is used, a large-diameter stent with a certain shape and a sufficient support force cannot be formed by second wires (i.e., thick wires) alone. Furthermore, even if diameters of the wires are the same, a large-diameter stent with a fixed wire-to-wire intersection formed by using for example a laser engraving technology has a much smaller support force, which cannot achieve requirements for aortic blood vessels according to the present application.

The expression "radial support force" of the stent as mentioned here refers to a force required to compress the stent along a diameter direction to have 85% of its original diameter, after it is fixed in a water bath at 37 ± 2 °C. The radial support force may be measured by a radial support force tester.

The stent may have a radial support force of greater than or equal to 100 N in the natural release state. The stent has a radial support force of 100 N to 600 N in the natural release state. The stent may have a radial support force of greater than or equal to 400 N in the axial maximum compression state. The stent has a radial support force of 400 N to 1000 N in the axial maximum compression state.

Along with the radial support force, the stent also requires a high liquid permeability to obtain an effect of effectively obstructing a tear of the intima of the blood vessel. This property may be represented by metal coverage. The stent 10 may have a metal coverage of at least 30% in the natural release state. The stent has a metal coverage of 30% to 90% in the natural release state. The stent 10 may have a metal coverage of 80% to 100% in the axial maximum compression state. The stent has a metal coverage of 80% to 95% in the axial maximum compression state.

The expression "metal coverage" of the stent as mentioned here refers to a metal coverage ratio per unit area, which is measured by electron microscope scanning. A sum of the metal coverage and a void ratio per unit area should be 100%.

According to an embodiment for reference only, the stent may have a radial support force of 200 N to 600 N and a metal coverage of 30% to 60% in the natural release state. The stent has a relatively low weaving density, and the stent also has a relatively low radial support force in the natural release state. The stent has a high axial compressible ratio, and the required radial support force and metal coverage may still be obtained after the stent is compressed. When the stent is placed, a relatively long segment of the stent is required to be released and then compressed, and this requires the blood vessel to have a longer straight segment. Therefore, the stent is not suitable for a site of the blood vessel such as the ascending aorta in which a turn is present.

According to another embodiment for reference only, the stent may have a radial support force of 100 N to 600 N and a metal coverage of 60% to 90%, preferably a metal coverage of 70% to 90%, in the natural release state. In the embodiment, the stent has a relatively high weaving density, and the stent also has a relatively high radial support force in the natural release state. In the embodiment, the stent has a low axial compressible ratio, and a certain radial support force and metal coverage may be obtained without compression after it is released. The stent is suitable to be placed in a site with a shorter straight segment in the blood vessel such as the ascending aorta.

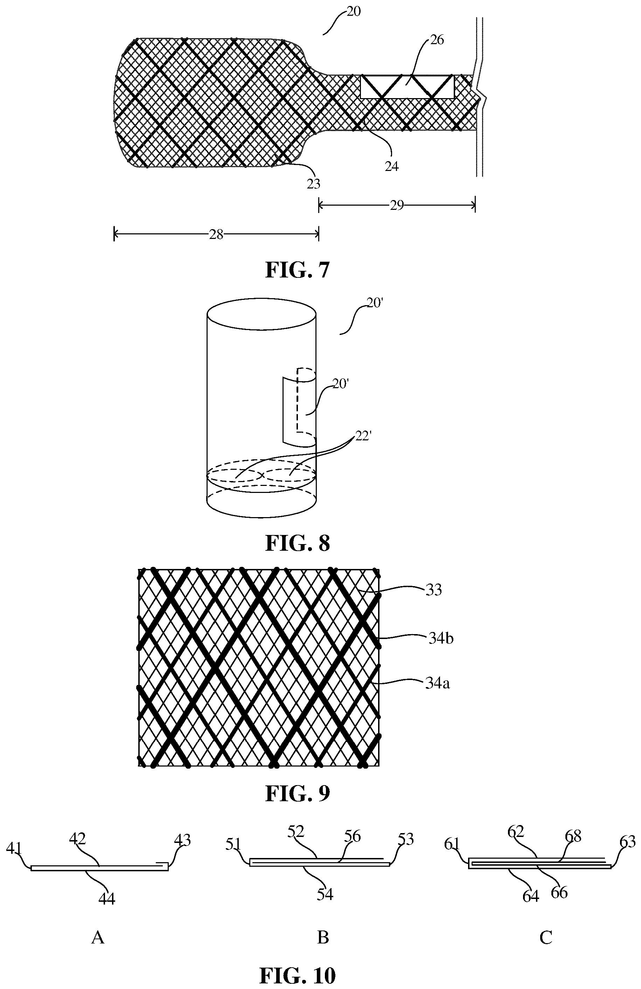

The stent of the embodiment has a relatively high metal coverage in the natural release state, and hinders blood flow to a certain extent. In order to maintain smooth blood flow at the branch artery, the stent of the embodiment has at least one sparse mesh area. With reference to FIG. 7, a stent 20 with a single-layer structure of the embodiment suitable to be placed at sites from the ascending aorta to the aortic arch is schematically shown. The stent 20 may be provided with multiple layers, such as 4 layers, to obtain a metal coverage in the above range. The stent 20 includes a segment 28 adapted to the ascending aorta and a segment 29 adapted to the aortic arch. The segment 28 adapted to the ascending aorta has a diameter ranging from about 38 mm to about 60 mm, and a diameter of the segment 29 adapted to the aortic arch is reduced to range from about 25 mm to about 35 mm. The segment 29 adapted to the aortic arch is provided with a sparse mesh area 26. Only the second wires (i.e., thick wires) 24 are arranged in the sparse mesh area 26. A part other than the sparse mesh area 26 is also provided with the first wires (i.e., thin wires) 23 in addition to the second wires. The sparse mesh area 26 is arranged at a site corresponding to a greater curvature of the aortic arch after the stent is released, and has an area larger than an opening of the branch vessel of the greater curvature in the artery. Typically, the sparse mesh area 26 may have a length of about 6 to 8 cm, and occupy about 1/3 arc length of the circumference of the segment 29 of the stent 20 with a central angle corresponding to about 120°.

According to another example, the stent (not shown) may be adapted to the whole arterial area from the ascending aorta to the abdominal aorta. After a segment of the ascending aorta, the stent is adapted to a segment which may extend from an area corresponding to the aortic arch to an area corresponding to the abdominal aorta, and the diameter of the stent may vary from about 25 mm ~ about 35 mm to about 20 mm ~ about 30 mm. The segment may include two sparse mesh areas formed of only the second wires, i.e., a first sparse mesh area corresponding to a branch artery (a brachiocephalic trunk artery, a common left neck artery, and a left subclavian artery) at the aortic arch, and a second sparse mesh area corresponding to a branch artery (a left renal artery, a right renal artery, a coeliac trunk artery, and a superior mesenteric artery) at the abdominal aorta. The first sparse mesh area may have a dimension in the embodiment as shown in FIG. 3. The second sparse mesh area may have a length of about 2 to 4 cm, and occupy about 1/2 arc length of the circumference of the segment with a central angle corresponding to about 180°.

The stent may be woven in a conventional manner, and then the first wires in a predetermined area are removed to form the sparse mesh area.

According to a further embodiment, at a part of the stent corresponding to the abdominal aorta which is the treatment site, the stent of the present application may be provided with two common iliac artery stent fixing parts configured to receive and fix left and right common iliac artery stents. With reference to FIG. 8, a stent of the embodiment is schematically shown. The stent 20' shown in FIG. 8 is suitable for a segment of the abdominal aorta and is provided with a sparse mesh area 27'. According to other embodiments, the stent 20' may not be provided with a sparse mesh area 27'. Common iliac artery stent fixing parts 22' arranged as two adjacent annular channels are provided at interior of a lower part of the stent 20'. For the sake of clarity, each fixing part 22' is shown as a plane in FIG. 4, and in fact, each fixing part 22' has a certain thickness. In some examples, each common iliac artery stent fixing part 22' may extend downward to a lower end of the stent 20'.

Each common iliac artery stent fixing part 22' is also formed by weaving the same first and second wires as the stent 20' (the first and second wires are not shown), and is integrally formed with the stent. Outer parts of the two annular channels are integrally woven with an inner wall of the stent 20'. The dimensions of inner diameters of the two annular channels are adapted to outer diameters of the left and right common iliac artery stents to be received and fixed, and are usually slightly less than the outer diameters of the left and right common iliac artery stents, so as to be able to fix the common iliac artery stents.

Of course, the embodiment in which the common iliac artery stent fixing parts are provided is also applicable to a stent without a sparse mesh area.

According to an embodiment, the stent of the present application may be formed by weaving three kinds of wires with different diameters. As shown in FIG. 9, a schematic partially enlarged view of a single layer of a stent of an example is shown. The stent is formed by weaving a kind of first wire 33 and two kinds of second wires 34a, 34b. Here the first wire 33 is a thin wire which may have a diameter of 50 µm to 150 µm; the second wires include a first thick wire 34a which may have a diameter of 150 µm to 300 µm and a second thick wire 34b which may have a diameter of 300 µm to 600 µm. Here the number of the first thick wire may be 6 to 12, the number of the second thick wire may be more than 4, but no more than 12 at most, and the remaining wires form the first wire. The embodiment may also have other variations. For example, the stent is formed by two kinds of first wires (for example, their diameters are in a range of 20 µm to 100 µm and a range of 100 µm to 150 µm, respectively) and a kind of second wire, or formed by two kinds of first wires and two kinds of second wires, but is not limited thereto.

A radial support force of the stent formed by three or more kinds of wires with different diameters is more uniform in the whole stent, and flexibility of the stent may also be enhanced.

The stent may be provided with multiple layers, preferably two layers, three layers, or four layers. According to a preferred embodiment for reference only, the stent with multiple layers may be formed by way of weaving a single-layer woven mesh in a return weaving manner. As shown in the parts A to C of FIG. 10, schematic cross-section views of woven layers of stents with two to four layers are shown. The part A of FIG. 10 shows a structure with two layers, and in this figure, an upper layer 42 of the structure is located at a side of the stent close to the interior of the stent, and a lower layer 44 of the stent is located at a side of the stent close to exterior (i.e., a side in contact with the wall of the blood vessel) of the stent. A smooth port is formed at a proximal end 41 of the stent due to the return weaving. At a distal end 43 of the structure, the lower layer 44 facing toward the exterior of the stent is woven in a return weaving manner by a certain distance, to wrap, inside the lower layer 44, an opened edge (burr) of a distal end of the upper layer 42 facing toward the interior of the stent,. Thus, a port with both smooth ends is formed. Similarly, in the part B of FIG. 10, an upper layer 52 is woven at a proximal end 51 in a return weaving manner to form a lower layer 54 and is further woven at a distal end 53 in a return weaving manner to form an intermediate layer 56. At the distal end 53, the lower layer 54 and the intermediate layer 56 are slightly longer than the upper layer 52, so that an opened edge of a distal end of the upper layer is located inside the stent. In this way, both ends of the stent are also smooth ports. The same principle is also applied to four layers in the part C of FIG. 10. At a distal end 63, two layers 62, 68 close to the interior of the stent are shorter than an edge formed by weaving two layers 64, 66 close to the exterior of the stent in a return weaving manner, while a proximal end 61 forms a lowermost layer 64 by weaving an uppermost layer 62 in a return weaving manner, to wrap two intermediate layers 68 and 66 therebetween. A variation may be possible that a distal end of the uppermost layer 62 or intermediate layer 68 is woven in a return weaving manner by a small segment, to wrap an opened edge of another layer. In this way, both ends of the stent are completely smooth ports.

Preferably, the stent of the present application is in the form of multiple layers, so that both ends (especially the proximal end) of the stent may form smooth ports, avoiding secondary damage to the blood vessel by an opened edge of the braid.

The stent of the present application is described in detail as above by way of examples. It should be understood by those skilled in the art that the above examples are intended to explain advantages of the stent of the present application, rather than limiting the scope of the present application, and features in an example may be applied to the stent of another example separately or in combination in an appropriate situation. Apparent variations and modifications made to the stent by those skilled in the art according to contents of the present application here fall within the scope of the present application, as long as they meet the concept of the present application.

Detailed solutions of the stent delivery system of the present application and the method for placing the stent into the blood vessel by using the system are explained by the above examples. Variations and modifications may be readily made by those skilled in the art based on the above contents, to be adapted to actual application requirements of the present application. These variations and modifications also fall within the scope of the present application.

## Claims

1. A stent delivery system (100, 100'), wherein the stent delivery system (100, 100') is used in an aorta, the stent delivery system (100, 100') comprising:
a delivery catheter (120), wherein the delivery catheter (120) comprises an outer catheter (130), an inner catheter (140) and a push rod (170) which are coaxially arranged sequentially from exterior to interior of the delivery catheter (120),
wherein the outer catheter (130) is provided with a proximal end (180) and a distal end (190), and is provided with a first hollow cavity (133) penetrating through the outer catheter (130),
the inner catheter (140) extends in the first hollow cavity (133) at the distal end (190) of the outer catheter (130), and is provided with a second hollow cavity (143) penetrating through the inner catheter (140),
the push rod (170) extends through the first hollow cavity (133) of the outer catheter (130) and the second hollow cavity (143) of the inner catheter (140), and extends out of a distal end of the inner catheter (140); and
a stent (150), wherein the stent (150) is a stent for use in the treatment of aortic lesion, and releasably maintained in the first hollow cavity (133) between the outer catheter (130) and the push rod (170) at the proximal end (180) of the outer catheter (130) in a delivery configuration,
wherein the delivery catheter (120) is configured so that the outer catheter (130), the inner catheter (140) and the push rod (170) are axially movable relative to one another, wherein a proximal end (154) of the stent (150) is removably constrained to a proximal end of the push rod (170), and a distal end (156) of the stent (150) is removably constrained to a proximal end of the inner catheter (140),
wherein the stent (150) is a stent which is at least partially compressible and extendable along an axial direction of the stent (150) in a natural release state, **characterized in that** the stent (150) has a radial support force of 100 N to 600 N and a metal coverage of 30% to 90% in the natural release state, and the stent (150) has a radial support force of 400 N to 1000 N and a metal coverage of 80% to 95% in a release and axial maximum compression state,
wherein the outer catheter (130) has an outer diameter of about 5 mm to about 10 mm,
wherein the stent (150) is formed by weaving at least two kinds of first wires (13, 23) and second wires (14, 24) with different diameters, and wherein each of the first wires (13, 23) has a diameter of 20 µm to 150 µm and each of the second wires (14, 24) has a diameter of 150 µm to 800 µm,
wherein the stent (150) is further provided with at least one sparse mesh area (26) formed only of the second wires (24), and the at least one sparse mesh area (26) is arranged at a site of a corresponding treatment site having a branch artery after the stent (150) is released,
wherein the stent (150) has a same diameter, or the stent (150) has a variable diameter, wherein the stent (150) has the diameter ranging from 20 mm to 60 mm.

2. The stent delivery system (100, 100') of claim 1, wherein the outer catheter (130) has the outer diameter of about 5 mm to about 7 mm.

3. The stent delivery system (100, 100') of claim 1, wherein the proximal end (154) of the stent (150) is removably constrained to the proximal end of the push rod (170) by a stopper, and the distal end (156) of the stent (150) is removably constrained to the proximal end of the inner catheter (140) by a stopper.

4. The stent delivery system (100, 100') of claim 1, wherein the second wires (14) comprise at least one first thick wire (34a) and at least one second thick wire (34b) with different diameters, and wherein the at least one first thick wire (34a) has a diameter of 150 µm to 300 µm, and the at least one second thick wire (34b) has a diameter of 300 µm to 600 µm.

5. The stent delivery system (100, 100') of claim 1 or 4, wherein the first wires (13) comprise at least one first thin wire and at least one second thin wire with different diameters, and wherein the at least one first thin wire has a diameter of 20 µm to 100 µm, and the at least one second thin wire has a diameter of 100 µm to 150 µm.

6. The stent delivery system (100, 100') of claim 4, wherein the stent (150) is formed by weaving 48 to 202 wires, wherein 6 to 24 first thick wires (34a) and 4 to 24 second thick wires (34b) of the 48 to 202 wires form the second wires (14), and remaining wires of the 48 to 202 wires form the first wires (13), provided that a sum of a number of the first thick wires (34a) and a number of the second thick wires (34b) is less than or equal to 32.

7. The stent delivery system (100, 100') of claim 5, wherein the stent (150) is formed by weaving 48 to 202 wires, wherein 4 to 32 wires of the 48 to 202 wires form the second wires (14), and remaining wires of the 48 to 202 wires form the first wires (13), and wherein the first wires (13) comprise 32 to 166 first thin wires and 32 to 166 second thin wires, provided that a sum of a number of the first thin wires and a number of the second thin wires is less than or equal to 198.

8. The stent delivery system (100, 100') of claim 1, wherein the stent (150) is used in an aorta comprising an abdominal aorta site, and the stent (150) is internally provided with two common iliac artery stent fixing parts (22') configured to fix left and right common iliac artery stents.

9. The stent delivery system (100, 100') of claim 8, wherein the common iliac artery stent fixing parts (22') are arranged inside the stent (150) and correspond to the abdominal aorta close to a bifurcation of left and right common iliac arteries, and wherein the two common iliac artery stent fixing parts (22') are configured as two annuluses tangent to each other and are integrally formed with an inner wall of the stent (150).

## Patentansprüche

1. Stentzuführsystem (100, 100'), wobei das Stentzuführsystem (100, 100') in einer Aorta verwendet wird, wobei das Stentzuführsystem (100, 100') umfasst:
einen Zuführkatheter (120), wobei der Zuführkatheter (120) einen Außenkatheter (130), einen Innenkatheter (140) und eine Schubstange (170) umfasst, die koaxial nacheinander von außen nach innen des Zuführkatheters (120) angeordnet sind,
wobei der Außenkatheter (130) mit einem proximalen Ende (180) und einem distalen Ende (190) versehen ist und mit einem ersten Hohlraum (133) versehen ist, der den Außenkatheter (130) durchdringt,
wobei sich der Innenkatheter (140) in dem ersten Hohlraum (133) an dem distalen Ende (190) des Außenkatheters (130) erstreckt und mit einem zweiten Hohlraum (143) versehen ist, der den Innenkatheter (140) durchdringt,
wobei sich die Schubstange (170) durch den ersten Hohlraum (133) des Außenkatheters (130) und den zweiten Hohlraum (143) des Innenkatheters (140) erstreckt und sich aus einem distalen Ende des Innenkatheters (140) heraus erstreckt; und
einen Stent (150), wobei der Stent (150) ein Stent zur Verwendung bei der Behandlung einer Aortenläsion ist und lösbar in dem ersten Hohlraum (133) zwischen dem Außenkatheter (130) und der Schubstange (170) an dem proximalen Ende (180) des Außenkatheters (130) in einer Zuführkonfiguration gehalten wird,
wobei der Zuführkatheter (120) so ausgebildet ist, dass der Außenkatheter (130), der Innenkatheter (140) und die Schubstange (170) axial relativ zueinander beweglich sind, wobei ein proximales Ende (154) des Stents (150) lösbar an einem proximalen Ende der Schubstange (170) fixiert ist und ein distales Ende (156) des Stents (150) lösbar an einem proximalen Ende des Innenkatheters (140) fixiert ist,
wobei der Stent (150) ein Stent ist, der in einem natürlichen Freisetzungszustand entlang einer axialen Richtung des Stents (150) zumindest teilweise komprimierbar und dehnbar ist, **dadurch gekennzeichnet, dass** der Stent (150) in dem natürlichen Freisetzungszustand eine radiale Stützkraft von 100 N bis 600 N und einen Metallbedeckungsgrad von 30 % bis 90 % aufweist, und der Stent (150) in einem Freisetzungs- und maximalen axialen Kompressionszustand eine radiale Stützkraft von 400 N bis 1000 N und einen Metallbedeckungsgrad von 80 % bis 95 % aufweist,
wobei der Außenkatheter (130) einen Außendurchmesser von etwa 5 mm bis etwa 10 mm aufweist,
wobei der Stent (150) durch Weben von mindestens zwei Arten von ersten Drähten (13, 23) und zweiten Drähten (14, 24) mit unterschiedlichen Durchmessern gebildet ist, und wobei jeder der ersten Drähte (13, 23) einen Durchmesser von 20 µm bis 150 µm aufweist und jeder der zweiten Drähte (14, 24) einen Durchmesser von 150 µm bis 800 µm aufweist,
wobei der Stent (150) ferner mit mindestens einem Bereich mit lockerem Geflecht (26) versehen ist, der nur aus den zweiten Drähten (24) gebildet ist, wobei der mindestens eine Bereich mit lockerem Geflecht (26) an einer Stelle einer entsprechenden Behandlungsstelle angeordnet ist, die eine Seitenastarterie aufweist, nachdem der Stent (150) freigesetzt wurde,
wobei der Stent (150) einen gleichen Durchmesser aufweist, oder der Stent (150) einen variablen Durchmesser aufweist, wobei der Stent (150) einen Durchmesser im Bereich von 20 mm bis 60 mm aufweist.

2. Stentzuführsystem (100, 100') nach Anspruch 1, wobei der Außenkatheter (130) den Außendurchmesser von etwa 5 mm bis etwa 7 mm aufweist.

3. Stentzuführsystem (100, 100') nach Anspruch 1, wobei das proximale Ende (154) des Stents (150) durch einen Anschlag lösbar an dem proximalen Ende der Schubstange (170) fixiert ist, und das distale Ende (156) des Stents (150) durch einen Anschlag lösbar an dem proximalen Ende des Innenkatheters (140) fixiert ist.

4. Stentzuführsystem (100, 100') nach Anspruch 1, wobei die zweiten Drähte (14) mindestens einen ersten dicken Draht (34a) und mindestens einen zweiten dicken Draht (34b) mit unterschiedlichen Durchmessern umfassen, und wobei der mindestens eine erste dicke Draht (34a) einen Durchmesser von 150 µm bis 300 µm aufweist, und der mindestens eine zweite dicke Draht (34b) einen Durchmesser von 300 µm bis 600 µm aufweist.

5. Stentzuführsystem (100, 100') nach Anspruch 1 oder 4, wobei die ersten Drähte (13) mindestens einen ersten dünnen Draht und mindestens einen zweiten dünnen Draht mit unterschiedlichen Durchmessern umfassen, und wobei der mindestens eine erste dünne Draht einen Durchmesser von 20 µm bis 100 µm aufweist, und der mindestens eine zweite dünne Draht einen Durchmesser von 100 µm bis 150 µm aufweist.

6. Stentzuführsystem (100, 100') nach Anspruch 4, wobei der Stent (150) durch Weben von 48 bis 202 Drähten gebildet ist, wobei 6 bis 24 erste dicke Drähte (34a) und 4 bis 24 zweite dicke Drähte (34b) der 48 bis 202 Drähte die zweiten Drähte (14) bilden, und die verbleibenden Drähte der 48 bis 202 Drähte die ersten Drähte (13) bilden, mit der Maßgabe, dass eine Summe aus einer Anzahl der ersten dicken Drähte (34a) und einer Anzahl der zweiten dicken Drähte (34b) kleiner oder gleich 32 ist.

7. Stentzuführsystem (100, 100') nach Anspruch 5, wobei der Stent (150) durch Weben von 48 bis 202 Drähten gebildet ist, wobei 4 bis 32 Drähte der 48 bis 202 Drähte die zweiten Drähte (14) bilden, und die verbleibenden Drähte der 48 bis 202 Drähte die ersten Drähte (13) bilden, und wobei die ersten Drähte (13) 32 bis 166 erste dünne Drähte und 32 bis 166 zweite dünne Drähte umfassen, mit der Maßgabe, dass eine Summe aus einer Anzahl der ersten dünnen Drähte und einer Anzahl der zweiten dünnen Drähte kleiner oder gleich 198 ist.

8. Stentzuführsystem (100, 100') nach Anspruch 1, wobei der Stent (150) in einer Aorta verwendet wird, die eine Stelle der Bauchaorta umfasst, und der Stent (150) innen mit zwei Befestigungsteilen für Stents der Arteria iliaca communis (22') versehen ist, die dazu ausgebildet sind, einen linken und einen rechten Stent der Arteria iliaca communis zu fixieren.

9. Stentzuführsystem (100, 100') nach Anspruch 8, wobei die Befestigungsteile für Stents der Arteria iliaca communis (22') im Inneren des Stents (150) angeordnet sind und einer Bifurkation der linken und rechten Arteria iliaca communis in der Bauchaorta nahe benachbart entsprechen, und wobei die beiden Befestigungsteile für Stents der Arteria iliaca communis (22') als zwei zueinander tangentiale Ringe ausgebildet sind und einstückig mit einer Innenwand des Stents (150) ausgebildet sind.

## Revendications

1. Système de pose d'endoprothèse (100, 100'), dans lequel le système de pose d'endoprothèse (100, 100') est utilisé dans une aorte, le système de pose d'endoprothèse (100, 100') comprenant :
un cathéter de pose (120), dans lequel le cathéter de pose (120) comprend un cathéter externe (130), un cathéter interne (140) et une tige de poussée (170) qui sont agencés coaxialement de manière séquentielle de l'extérieur vers l'intérieur du cathéter de pose (120),
dans lequel le cathéter externe (130) est pourvu d'une extrémité proximale (180) et d'une extrémité distale (190), et est pourvu d'une première cavité creuse (133) traversant le cathéter externe (130),
le cathéter interne (140) s'étend dans la première cavité creuse (133) au niveau de l'extrémité distale (190) du cathéter externe (130), et est pourvu d'une seconde cavité creuse (143) traversant le cathéter interne (140),
la tige de poussée (170) s'étend à travers la première cavité creuse (133) du cathéter externe (130) et la seconde cavité creuse (143) du cathéter interne (140), et s'étend hors d'une extrémité distale du cathéter interne (140) ; et
une endoprothèse (150), dans laquelle l'endoprothèse (150) est une endoprothèse destinée à être utilisée dans le traitement d'une lésion aortique, et est maintenue de manière libérable dans la première cavité creuse (133) entre le cathéter externe (130) et la tige de poussée (170) au niveau de l'extrémité proximale (180) du cathéter externe (130) dans une configuration de pose,
dans lequel le cathéter de pose (120) est configuré de sorte que le cathéter externe (130), le cathéter interne (140) et la tige de poussée (170) sont mobiles axialement les uns par rapport aux autres, dans lequel une extrémité proximale (154) de l'endoprothèse (150) est contrainte de manière amovible à une extrémité proximale de la tige de poussée (170), et une extrémité distale (156) de l'endoprothèse (150) est contrainte de manière amovible à une extrémité proximale du cathéter interne (140),
dans lequel l'endoprothèse (150) est une endoprothèse qui est au moins partiellement compressible et extensible selon une direction axiale de l'endoprothèse (150) dans un état de libération naturelle, **caractérisé en ce que** l'endoprothèse (150) présente une force de support radial de 100 N à 600 N et un taux de couverture métallique de 30 % à 90 % dans l'état de libération naturelle, et l'endoprothèse (150) présente une force de support radial de 400 N à 1000 N et un taux de couverture métallique de 80 % à 95 % dans un état de libération et de compression axiale maximale,
dans lequel le cathéter externe (130) présente un diamètre extérieur d'environ 5 mm à environ 10 mm,
dans lequel l'endoprothèse (150) est formée par tissage d'au moins deux sortes de premiers fils (13, 23) et de seconds fils (14, 24) de diamètres différents, et dans lequel chacun des premiers fils (13, 23) présente un diamètre de 20 µm à 150 µm et chacun des seconds fils (14, 24) présente un diamètre de 150 µm à 800 µm,
dans lequel l'endoprothèse (150) est en outre pourvue d'au moins une zone de maille clairsemée (26) formée uniquement des seconds fils (24), et l'au moins une zone de maille clairsemée (26) est disposée au niveau d'un site d'un site de traitement correspondant comportant une artère collatérale après la libération de l'endoprothèse (150),
dans lequel l'endoprothèse (150) présente un même diamètre, ou l'endoprothèse (150) présente un diamètre variable, dans lequel l'endoprothèse (150) présente un diamètre allant de 20 mm à 60 mm.

2. Système de pose d'endoprothèse (100, 100') selon la revendication 1, dans lequel le cathéter externe (130) présente le diamètre extérieur d'environ 5 mm à environ 7 mm.

3. Système de pose d'endoprothèse (100, 100') selon la revendication 1, dans lequel l'extrémité proximale (154) de l'endoprothèse (150) est contrainte de manière amovible à l'extrémité proximale de la tige de poussée (170) par une butée, et l'extrémité distale (156) de l'endoprothèse (150) est contrainte de manière amovible à l'extrémité proximale du cathéter interne (140) par une butée.

4. Système de pose d'endoprothèse (100, 100') selon la revendication 1, dans lequel les seconds fils (14) comprennent au moins un premier fil épais (34a) et au moins un second fil épais (34b) de diamètres différents, et dans lequel l'au moins un premier fil épais (34a) présente un diamètre de 150 µm à 300 µm, et l'au moins un second fil épais (34b) présente un diamètre de 300 µm à 600 µm.

5. Système de pose d'endoprothèse (100, 100') selon la revendication 1 ou 4, dans lequel les premiers fils (13) comprennent au moins un premier fil fin et au moins un second fil fin de diamètres différents, et dans lequel l'au moins un premier fil fin présente un diamètre de 20 µm à 100 µm, et l'au moins un second fil fin présente un diamètre de 100 µm à 150 µm.

6. Système de pose d'endoprothèse (100, 100') selon la revendication 4, dans lequel l'endoprothèse (150) est formée par tissage de 48 à 202 fils, dans lequel 6 à 24 premiers fils épais (34a) et 4 à 24 seconds fils épais (34b) parmi les 48 à 202 fils forment les seconds fils (14), et les fils restants parmi les 48 à 202 fils forment les premiers fils (13), à condition qu'une somme d'un nombre des premiers fils épais (34a) et d'un nombre des seconds fils épais (34b) soit inférieure ou égale à 32.

7. Système de pose d'endoprothèse (100, 100') selon la revendication 5, dans lequel l'endoprothèse (150) est formée par tissage de 48 à 202 fils, dans lequel 4 à 32 fils parmi les 48 à 202 fils forment les seconds fils (14), et les fils restants parmi les 48 à 202 fils forment les premiers fils (13), et dans lequel les premiers fils (13) comprennent 32 à 166 premiers fils fins et 32 à 166 seconds fils fins, à condition qu'une somme d'un nombre des premiers fils fins et d'un nombre des seconds fils fins soit inférieure ou égale à 198.

8. Système de pose d'endoprothèse (100, 100') selon la revendication 1, dans lequel l'endoprothèse (150) est utilisée dans une aorte comprenant un site d'aorte abdominale, et l'endoprothèse (150) est pourvue intérieurement de deux parties de fixation d'endoprothèse d'artère iliaque commune (22') configurées pour fixer des endoprothèses d'artère iliaque commune gauche et droite.

9. Système de pose d'endoprothèse (100, 100') selon la revendication 8, dans lequel les parties de fixation d'endoprothèse d'artère iliaque commune (22') sont disposées à l'intérieur de l'endoprothèse (150) et correspondent à l'aorte abdominale à proximité d'une bifurcation des artères iliaques communes gauche et droite, et dans lequel les deux parties de fixation d'endoprothèse d'artère iliaque commune (22') sont configurées sous forme de deux anneaux tangents l'un à l'autre et sont formées d'un seul tenant avec une paroi interne de l'endoprothèse (150).
